(19) Europäisches Patentamt
European Patent Office
Office européen des brevets

(11) **EP 2 741 656 B1**

(12) **FASCICULE DE BREVET EUROPEEN**

(45) Date de publication et mention de la délivrance du brevet:
**24.12.2025 Bulletin 2025/52**

(21) Numéro de dépôt: **11757906.0**

(22) Date de dépôt: **09.08.2011**

(51) Classification Internationale des Brevets (IPC):
*A61B 3/02* (2006.01)   *A61B 3/032* (2006.01)
*A61B 3/00* (2006.01)   *A61B 3/113* (2006.01)
*A61B 3/06* (2006.01)   *A61B 3/024* (2006.01)
*A61B 3/08* (2006.01)   *G02C 7/10* (2006.01)

(52) Classification Coopérative des Brevets (CPC):
**A61B 3/032; A61B 3/02;** A61B 3/0033; A61B 3/005; A61B 3/022; A61B 3/024; A61B 3/063; A61B 3/066; A61B 3/08; A61B 3/113; G02C 7/10

(86) Numéro de dépôt international:
**PCT/FR2011/000462**

(87) Numéro de publication internationale:
**WO 2013/021102 (14.02.2013 Gazette 2013/07)**

(54) **DISPOSITIF POUR DÉTERMINER UN GROUPE D'ÉQUIPEMENTS D'AIDE A LA VISION ADAPTE A UN INDIVIDU**

VORRICHTUNG ZUR BESTIMMUNG EINER FÜR EINE PERSON GEEIGNETEN GRUPPE VON SEHHILFEN

DEVICE FOR DETERMINING A GROUP OF VISION AIDS SUITABLE FOR A PERSON

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**18.06.2014 Bulletin 2014/25**

(73) Titulaire: **Essilor International**
**94220 Charenton-le-Pont (FR)**

(72) Inventeurs:
• **SCHERLEN, Anne-Catherine**
**F-94220 Charenton-le-Pont (FR)**
• **VOILLEMIN, Pascal**
**F-94220 Charenton-le-Pont (FR)**

(74) Mandataire: **Jacobacci Coralis Harle**
**32, rue de l'Arcade**
**75008 Paris (FR)**

(56) Documents cités:
**EP-A1- 1 333 393**      **EP-A1- 1 336 924**
**EP-A1- 1 892 660**      **WO-A1-01/34020**
**WO-A1-2006/010611**     **WO-A1-2008/089995**
**US-A1- 2004 174 499**

**Description**

DOMAINE TECHNIQUE AUQUEL SE RAPPORTE L'INVENTION

La présente invention concerne un dispositif pour déterminer un groupe d'au moins un équipement d'aide à la vision adapté à la vision d'un individu.

ARRIERE-PLAN TECHNOLOGIQUE

Il existe de très nombreux équipements d'aide à la vision présentant des caractéristiques extrêmement différentes, tant du point de vue du type d'aide à la vision apportée, par exemple amélioration de l'acuité visuelle, du contraste, ou diminution de l'éblouissement, que du point de vue du type d'utilisations possibles de cet équipement, par exemple utilisation en intérieur ou en extérieur, utilisation pour lire, pour écrire, pour regarder au loin...

La détermination d'un ou plusieurs d'équipement visuel le mieux adapté à un individu en fonction de sa vision et de l'utilisation qu'il souhaite faire de l'équipement requiert ainsi actuellement un processus relativement long et pénible tant pour l'opticien que pour l'individu.

On connait du document EP1336924 A1 un dispositif pour sélectionner une lentille adaptée à la vision d'un individu.

Le document EP1333393 A1 divulgue de prendre en compte les caractéristiques de vision, les conditions ergonomiques d'utilisation et les utilisations souhaitées pour la sélection d'une aide à la vision.

OBJET DE L'INVENTION

Afin de remédier à l'inconvénient précité de l'état de la technique, la présente invention propose un dispositif permettant de déterminer simplement et efficacement au moins un équipement spécifiquement adapté aux besoins et aux souhaits d'un individu.

Plus particulièrement, on propose selon l'invention un dispositif pour déterminer un groupe d'au moins un équipement d'aide à la vision adapté à la vision d'un individu tel que décrit dans la revendication 1.

Ainsi, un même dispositif est adapté, d'une part, à effectuer les tests de vision de l'individu permettant de déterminer précisément les caractéristiques techniques de l'équipement adapté à améliorer la vision de l'individu et, d'autre part, à enregistrer les souhaits de l'individu en matière d'utilisation de l'équipement.

On entend ici par tests de vision des tests visant à déterminer les caractéristiques de fonctionnement aussi bien optiques, physiques, mécaniques, physiologiques ou nerveuses des yeux de l'individu.

On entend ici par utilisation de l'équipement aussi bien des activités souhaitées par l'utilisateur, par exemple lire ou voir de loin ou des conditions d'utilisations de l'équipement, par exemple avoir les mains libres, utiliser l'équipement pendant de longues durées...

Enfin, le dispositif selon l'invention est programmé pour sélectionner un groupe d'équipement adapté à l'individu en croisant les deux types d'informations recueillies.

D'autres caractéristiques non limitatives et avantageuses du dispositif conforme à l'invention sont énoncées dans les revendications 2 à 7. conforme à l'invention sont énoncées dans les revendications 2 à 10.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 est une vue schématique du dispositif selon l'invention,
- la figure 2 est une vue schématique d'un mode de fonctionnement possible du dispositif de la figure 1,
- la figure 3 est une représentation schématique d'un test d'acuité visuelle affiché sur le dispositif de la figure 1,
- la figure 4 est une représentation schématique d'un test de contraste affiché sur le dispositif de la figure 1.

Le dispositif de détermination d'un groupe d'au moins un équipement visuel selon l'invention peut être utilisé pour tout individu, quelles que soient les caractéristiques de sa vision.

Il peut par exemple permettre de déterminer si l'équipement le mieux adapté à un individu fait partie du groupe des lunettes munies de lentilles correctrices simple foyer, double foyer ou progressives.

Cependant, ce dispositif est particulièrement avantageux pour déterminer un groupe d'équipements destiné à aider une personne malvoyante.

En effet, la vision des personnes malvoyantes peut être altérée par de très nombreux facteurs. Il peut s'agir par exemple d'une baisse de l'acuité visuelle, d'une diminution de la perception des contrastes ou des couleurs, d'une diminution de la motricité et/ou du champ de vision des yeux de cette personne.

En outre, l'altération de la vision de ces personnes étant particulièrement importante, il n'est pas toujours possible de leur proposer un équipement d'aide à la vision adapté à être utilisé dans toutes les situations.

Le dispositif selon l'invention permet alors de manière simple et rapide d'évaluer différents aspects de la vision de l'individu ainsi que les utilisations envisagées par l'individu de l'équipement d'aide à la vision, pour enfin sélectionner un groupe d'équipements réalisant le meilleur compromis entre les caractéristiques techniques nécessaires à la correction des défauts de vision de l'individu et les caractéristiques ergonomiques et pratiques nécessaires pour les utilisations envisagées en priorité par cet individu.

Plus précisément, il existe plusieurs centaines d'équipements d'aide à la vision pour personnes malvoyantes présentant des caractéristiques techniques, ergonomiques et pratiques différentes.

L'ensemble des équipements d'aide à la vision peut être segmenté en sept groupes d'équipement d'aide à la vision :

- les loupes,
- les lunettes microscopiques (ou lunettes à forte puissance),
- les systèmes téléscopiques de Galilée et Kepler,
- les aides électroniques portables,
- les aides électroniques du type télé-agrandisseurs,
- les lampes,
- les filtres.

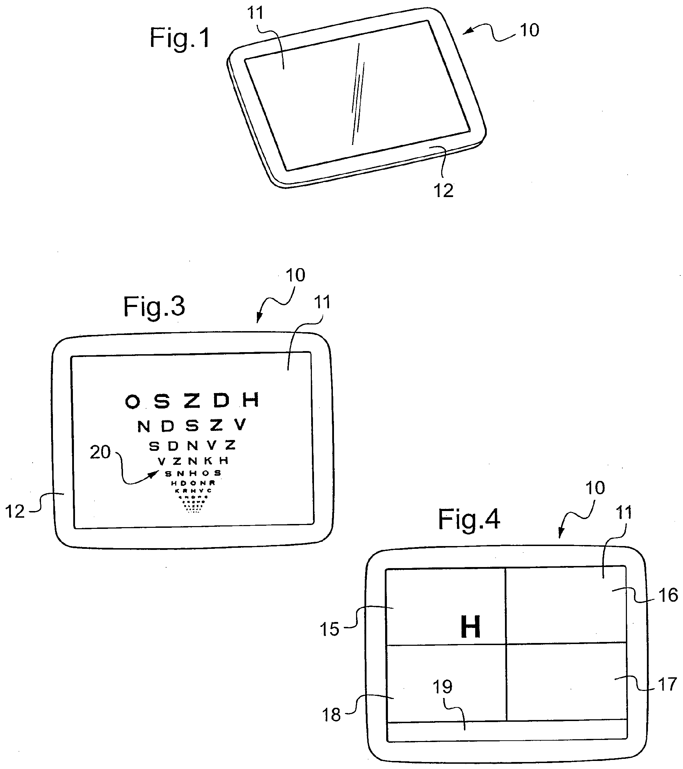

Sur la figure 1, on a représenté un exemple de réalisation possible pour le dispositif selon l'invention.

Ce dispositif se présente ici sous la forme d'une tablette 10 tactile comportant un écran d'affichage tactile 11 et un cadre 12 l'entourant.

Cette tablette 10 peut être tenue par l'individu dont la vision est évaluée, posée sur une table ou intégrée à une machine plus complexe.

La tablette 10 comporte des moyens informatiques ainsi que

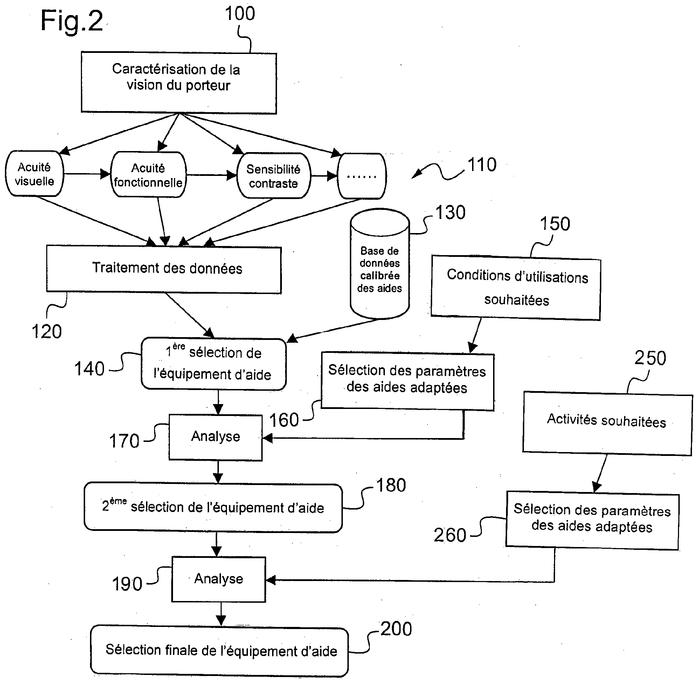

- des moyens pour déterminer au moins une caractéristique de la vision dudit individu dans une étape a) (bloc 100 de la figure 2) ,
- des moyens pour déterminer au moins une utilisation du groupe d'équipements d'aide à la vision souhaitée par l'individu dans une étape b) (bloc 150 de la figure 2),
- des moyens pour déterminer ledit groupe d'équipements d'aide à la vision en fonction de la caractéristique de la vision et de l'utilisation souhaitée par l'individu dans une étape c) (bloc 180 de la figure 2).

Les moyens informatiques sont programmés pour commander les moyens précités.

Le fonctionnement général de ce dispositif est représenté schématiquement sur la figure 2.

Dans l'étape a) représentée par le bloc 100 de la figure 2, le dispositif selon l'invention caractérise la vision du porteur. Pour cela, une série de tests représentés par les blocs 110 sont réalisés grâce au dispositif 10.

Les résultats de ces tests sont traités pas les moyens informatiques pour déterminer les caractéristiques optiques de l'équipement adapté à l'individu testé (bloc 120 de la figure 2).

Ensuite, ces caractéristiques optiques sont confrontées (bloc 140 de la figure 2) aux caractéristiques des équipements disponibles qui sont regroupées dans une base de données 130 pour sélectionner un premier ensemble d'équipements adaptés (étape c1)).

Dans l'étape b) représentée par les blocs 150 et 250 sur la figure 2, des informations concernant l'utilisation de l'équipement souhaitée par l'individu sont recueillies par le dispositif 10. Ces informations concernent d'une part les conditions d'utilisation ergonomique et pratique de l'équipement (bloc 150) et les activités souhaitées par l'individu (bloc 160). Ces informations sont traitées par les moyens informatiques pour en déduire d'une part les caractéristiques ergonomiques et pratiques des équipements adaptés à l'individu interrogé (bloc 160 de la figure 2) et d'autre part les équipements adaptés à ces activités (bloc 260).

Enfin, dans l'étape c2), les résultats de la première sélection et du traitement des informations recueillies sur les conditions d'utilisation de l'équipement sont confrontés dans une étape d'analyse (bloc 170), afin de sélectionner les équipements adaptés à l'utilisation envisagée par l'individu parmi ceux de l'ensemble présentant les caractéristiques optiques susceptibles d'aider l'individu (bloc 180).

Dans l'étape c3), les résultats de la deuxième sélection et du traitement des informations recueillies sur les activités souhaitées par l'individu sont confrontés dans une étape d'analyse (bloc 190), afin de sélectionner les équipements adaptés aux activités envisagées par l'individu parmi ceux de la deuxième sélection (bloc 200).

Etape a)

A l'étape a), l'une au moins des caractéristiques suivantes de la vision de la personne malvoyante est déterminée : acuité visuelle, sensibilité au contraste, étendue du champ de vision, éblouissement, oculomotricité.

A cet effet, les moyens informatiques de la tablette sont programmés pour afficher sur l'écran 11 une série de tests permettant d'évaluer les caractéristiques de la vision de l'individu.

Chaque test comprend d'une part l'affichage d'un signe sur l'écran et d'autre part, la réception d'un signal émis par l'individu. Les moyens informatiques de la tablette sont programmés pour interpréter ce signal et en déduire le résultat du test.

Les signes affichés sur la tablette correspondent par exemple à une lettre d'un alphabet connu de l'individu ou à un texte rédigé dans une langue parlée par l'individu.

Comme expliqué en détails plus loin, l'enchaînement des tests est déterminé par les moyens informatiques de la tablette en fonction du résultat des tests antérieurs.

La tablette 10 étant tactile, le signal émis par l'individu correspond par exemple au toucher d'une zone particulière de la tablette.

Cependant, on peut également envisager d'autres modes de réalisation, dans lesquels le signal serait un signal sonore émis par l'individu, par exemple lorsqu'il lit une lettre ou un texte sur la tablette.

Dans la suite, un exemple de test possible sera décrit pour différentes caractéristiques de la vision qui peuvent être évaluées. D'autres tests peuvent évidemment être envisagés et d'autres caractéristiques de la vision peuvent être testées.

### Test d'acuité visuelle

Le test d'acuité visuelle est par exemple réalisé par l'affichage de douze lignes de lettres 20 comme représenté sur la figure 3. Chaque ligne de lettre est composée de cinq lettres majuscules. Les lettres utilisées sont les lettres appelées lettres de Sloan, c'est-à-dire S, O, C, D, K, V, R, H, N, Z, comme cela est décrit dans l'ouvrage « Borish's Clinical Refraction », de William J. Benjamin, publié en 2006 par Butterworth-Heinemann/Elsevier.

Ces lettres sont en effet facilement reconnaissables. Dans une langue différente du français, et notamment dans une langue utilisant un alphabet différent, on peut également prendre toutes les lettres de l'alphabet ou un groupe de lettres différent.

Il est possible à l'aide d'un bouton affiché sur l'écran de distribuer les lettres sur l'écran de manière aléatoire ou de manière prédéterminée pour effectuer des mesures d'acuités visuelles dans différentes conditions, par exemple en conditions de vision monoculaire ou binoculaire, pour effectuer une simple vérification de l'acuité ou dans le cas de plusieurs tests successifs d'un même individu. Cela permet d'éviter la mémorisation des tests par l'individu.

Sur chaque ligne, la taille des lettres correspond à un angle visuel de discrimination, c'est-à-dire à une acuité visuelle déterminée.

La taille des lettres diminue de la première à la dernière ligne.

Ceci signifie que si l'individu arrive à lire les lettres d'une ligne donnée, son acuité visuelle est supérieure ou égale à l'angle visuel de discrimination correspondant à la taille des lettres de cette ligne.

La taille des lettres est par exemple calibrée pour une distance de lecture de 40 cm. La plage d'acuité visuelle testée dépend ici en partie des caractéristiques de résolution de l'écran tactile utilisé. Il est possible avec les écrans actuels d'afficher des lignes de lettres permettant de tester les acuités visuelles comprises entre 5/10 à 1/25. De préférence, le pas d'acuité visuelle entre chaque ligne est constant, ce qui permet une mesure régulière et fine quelle que soit la plage d'acuités visuelles testée. En pratique ici, les lettres de chaque ligne sont plus hautes que celles de la ligne inférieure d'un coefficient multiplicatif constant égal à la racine cubique de 2. La progression des tailles de lettres est ainsi logarithmique.

Pour une ligne donnée, l'espacement entre deux lettres est égal à la taille de la lettre de cette ligne.

L'espacement entre deux lignes successives est égal à la taille de la ligne du dessous, c'est-à-dire la plus petite taille. Le contraste des lettres affichées est de 100%.

L'individu doit ici pointer la dernière ligne qu'il peut lire en touchant l'écran tactile au niveau de cette ligne. La tablette enregistre alors l'acuité visuelle correspondant à cette ligne comme étant l'acuité visuelle de l'individu.

Par exemple, si l'individu peut lire la cinquième ligne de lettres qui mesurent 0,582 centimètre, mais aucune ligne de lettres située en dessous de cette cinquième ligne, les moyens informatiques de la tablette en déduisent que l'acuité visuelle de l'individu est de 1/10.

Les équipements d'aide à la vision qui pourront aider l'individu doivent alors présenter un grossissement au moins égal à 6.

Cette action fait en outre apparaître le test suivant.

### Test d'acuité fonctionnelle

Le test d'acuité fonctionnelle permet de déterminer l'acuité en lecture du porteur et d'obtenir une valeur plus précise du grossissement utile de l'équipement d'aide à la vision adapté à l'individu.

Les caractéristiques de la vision de l'individu liées à la lecture d'un texte dépendent des capacités d'identification et des capacités d'oculomotricité de l'individu en plus de son acuité visuelle.

Si ces capacités sont altérées, le grossissement de l'équipement d'aide à la vision adapté à l'individu devra être augmenté en conséquence.

Le test d'acuité fonctionnelle permet également de déterminer le grossissement utile de l'équipement d'aide à la vision dans des conditions plus proches des conditions de la vie réelle de l'individu.

Dans l'exemple de test d'acuité fonctionnelle décrit ici, les moyens informatiques de la tablette sont programmés pour afficher sur l'écran une phrase constituée de 3 lignes, présentant donc deux retours à la ligne.

Le retour à la ligne peut constituer des difficultés pour des individus dont la vision est particulièrement dégradée (perte de repères, saut de lignes, ...).

Les mots des phrases sélectionnées sont de préférence sélectionnés pour être des mots simples à comprendre. Ces phrases proviennent par exemple d'un texte bien connu du type contes ou fables.

Les mots composants ces phrases peuvent également être choisis en fonction de leur occurrence dans la langue considérée. Ces occurrences sont déterminées par des études scientifiques. En français, on trouve les résultats d'une telle étude à l'adresse internet suivante : http://www.lexique.org.

Il s'agit de préférence des mots les plus fréquents dans la langue considérée.

Chaque phrase comprend de préférence entre 10 et 15 mots présentant une distribution homogène de mots courts, comportant 2 lettres ou moins, de mots de longueurs moyennes, comportant entre 3 et 5 lettres et de mots longs, comportant plus de 5 lettres.

Une seule phrase est affichée centrée sur l'écran 11. La taille des lettres de la première phrase affichée après le test d'acuité visuelle, est une taille de lettre correspondant à une acuité inférieure à celle déterminée pour l'individu lors du test d'acuité visuelle, c'est-à-dire à une taille de lettre plus grande, pour faciliter la lecture de la première phrase.

L'individu fait ensuite afficher d'autres phrases avec des tailles de lettres décroissantes ou croissantes en touchant l'écran. La taille des lettres des phrases suit une progression logarithmique similaire à celle des tailles de lettres des différentes lignes de lettres affichées lors du test d'acuité visuelle.

L'individu ou l'opérateur valide la phrase présentant la taille de lettres la plus petite pour laquelle la lecture est fluide et sans fautes.

La taille des lettres de la phrase validée correspond à une acuité visuelle qui est l'acuité de lecture. Si cette acuité fonctionnelle est inférieure à l'acuité visuelle du porteur, la valeur la plus faible des deux est retenue par les moyens informatiques de la tablette.

Test de contraste

Le test de contraste permet de mesurer la sensibilité de l'individu aux contrastes. Le contraste est défini comme une capacité à pouvoir distinguer un objet de son fond. Le contraste est défini en % ou en Log. Il est par exemple calculé selon la formule du contraste de Michelson:

$$(LF - LO) / (LF + LO),$$

, où LF est la luminance du fond et LO la luminance de l'objet.

Lorsque la luminance est codée sur un octet, la luminance du blanc est égale à 255 et la luminance du noir est égale à 0.

Un contraste de 100% correspond à une lettre noire sur un fond blanc. Le test de contraste est effectué de manière ludique, simple et rapide.

A cet effet, on affiche successivement à l'écran des lettres choisies parmi les lettres de Sloan, sur un fond blanc. Les lettres présentent une taille bien supérieure à la taille correspondant à l'acuité visuelle de l'individu, par exemple une hauteur de quatre centimètres.

On affiche par exemple des lettres présentant huit contrastes prédéfinis et égaux aux valeurs suivantes : 100%, 50%, 25%, 10%, 5%, 2,5%, 1,25%, et 0,6%. Les contrastes affichés peuvent également être choisi avec un pas constant sur une échelle logarithmique.

L'écran de l'affichage est divisé en 5 zones représentées schématiquement sur la figure 4.

Les quadrants 15, 16, 17, 18 correspondent aux zones d'affichage possibles de la lettre, ici un H affiché dans le quadrant supérieur gauche. La zone 19 est une zone formant un bouton de réponse « pas de lettres perçues ». L'application affiche une lettre choisie aléatoirement parmi les lettres de Sloan dans l'un des quadrants 15, 16, 17, 18 choisi également aléatoirement.

Le contraste de la lettre affichée diminue de préférence progressivement.

Lorsqu'il voit une lettre affichée, l'individu doit lire la lettre à haute voix et toucher le quadrant dans lequel il voit apparaître la lettre.

Lorsque l'individu ne voit pas de lettre affichée, il a la possibilité de l'indiquer en touchant la zone 19 de l'écran.

La réponse est validée si l'individu pointe bien la lettre affichée.

Si l'individu touche une autre zone de l'écran, les moyens informatiques considèrent que l'individu n'a pas perçu la lettre affichée.

Lorsqu'une lettre apparaît dans un quadrant et que l'individu indique qu'il ne voit aucune lettre ou se trompe de quadrant, ceci signifie que le contraste maximal perçu par l'individu est compris entre les valeurs de contraste de la lettre précédente et de la lettre affichée.

On peut envisager d'inclure dans la série de lettres affichées, toutes les deux lettres affichées par exemple, une lettre présentant un contraste aléatoire.

Ceci rend le test plus ludique et évite de mettre l'individu en échec.

Les moyens informatiques de la tablette enregistrent comme seuil de contraste la valeur du contraste de la dernière lettre vue. Le seuil de contraste est définitivement validé lorsque l'individu a indiqué trois fois aux moyens informatiques de la tablette le même seuil de contraste.

De préférence, les trois premières lettres affichées présentent des contrastes de 100%, 50% et 25% et sont facilement identifiables par l'individu.

Cela constitue une phase d'entraînement et de mise en confiance de l'individu. Elle permet de vérifier que l'individu a bien compris les instructions de l'opérateur. Les résultats de cette phase d'entraînement ne sont pas pris en compte dans le calcul de la sensibilité aux contrastes.

Test de pointage

Le test de pointage permet de caractériser la qualité de la coordination œil-main de l'individu. Des personnes malvoyantes peuvent fréquemment présenter des défauts de coordination qui les mènent à pointer à côté d'un objet ciblé. Cette caractéristique liée à la vision de l'individu joue un rôle important dans le choix de l'équipement d'aide à la vision.

Pour ce test, les moyens informatiques sont programmés pour afficher des croix noires sur fond blanc, présentant un contraste de 100%, disposées de manière aléatoire sur l'écran. La taille de la croix est suffisamment grande pour rester visible par l'individu en fonction de l'acuité visuelle mesurée initialement.

Si l'acuité visuelle est inférieure ou égale à 0,8/10, la taille de la croix est égale à cinq centimètres.

Si l'acuité visuelle est comprise entre 0,8/10 et 2,5/10, la taille de la croix est égale à 3 centimètres.

Si l'acuité visuelle est supérieure ou égale à 2,5/10, la taille de la croix est égale à 1,7 centimètres.

Un test de pointage comporte l'affichage d'une série de dix croix successives sur l'écran. On peut également envisager d'afficher plusieurs séries de croix selon que l'on veuille évaluer la coordination en vision monoculaire ou en vision binoculaire.

On demande alors à l'individu de pointer le centre de la croix affichée le plus vite possible. On relève, pour chaque croix affichée, le temps mis par l'individu pour pointer, égal au temps entre l'affichage de la croix et le moment où l'individu touche l'écran et l'écart de pointage entre le centre de la croix, de coordonnées X_croix, Y_croix et la zone de pointage du doigt, de coordonnées X_doigt, Y_doigt.

Après chaque pointage, une nouvelle croix est affichée sur l'écran à une position aléatoire.

Pour chaque test, on défini la moyenne de l'écart entre la position du centre de la croix et la position du point touché par l'individu.

Une amplitude et une direction de l'écart sont calculées.

Une phase d'entraînement peut être ajoutée au test. Celui-ci comporte alors l'affichage de 13 croix. Les résultats des 3 premiers essais ne sont pas comptés.

## Test d'éblouissement

Le test d'éblouissement permet de déterminer la sensibilité à la lumière de l'individu. La sensibilité est caractérisée par trois paramètres :

- l'intensité lumineuse minimale mesurée en lux engendrant une gêne,
- la baisse d'acuité visuelle et de sensibilité aux contrastes associées à cette intensité lumineuse,
- le temps de récupération de l'acuité visuelle et de la sensibilité aux contrastes après une exposition prolongée à la lumière.

Pour cela le dispositif est constitué d'un affichage de lettres d'acuité variable et de contraste variable couplé à une source de lumière. La source de lumière comprend un variateur permettant de diffuser un flux lumineux compris entre 0 et 7000 lux. Le sujet se trouve entre 40 et 60 centimètres de la source lumineuse. La source lumineuse peut être soit centrale et ponctuelle, soit périphérique, par exemple annulaire ou circulaire.

On affiche initialement un ensemble de lettres, par exemple deux ou trois lettres, dont la taille et le contraste permettent à l'individu de les distinguer clairement d'après les résultats des tests d'acuité visuelle et de contraste précédents.

On augmente ensuite progressivement le flux lumineux diffusé par la source lumineuse jusqu'à ce que l'individu signale une gêne.

On réalise à nouveau alors un test d'acuité visuelle et un test de contraste afin de déterminer s'il y a une diminution de l'acuité visuelle et/ou une perte de contraste.

Si cela est le cas, on détermine la nouvelle acuité visuelle et la nouvelle perception du contraste avec ce flux lumineux.

Cette étape permet ainsi de quantifier l'éblouissement en déterminant le flux lumineux maximal et les conséquences de ce flux lumineux sur la vision de l'individu.

Dans une seconde étape, on illumine l'individu par un flux lumineux maximal et on mesure le temps nécessaire pour que la personne retrouve son acuité visuelle initiale.

Ce temps appelé par exemple « temps de récupération », est mesuré soit avec un chronomètre contrôlé par un opérateur ou activé par une touche sur la tablette. Le temps de récupération est alors mesuré entre l'instant où l'individu touche une première fois l'écran tactile de la tablette, au moment de l'illumination et l'instant où l'individu touche à nouveau l'écran pour indiquer qu'il a récupéré son acuité visuelle.

En pratique, si ce temps est inférieur à 30 secondes, il n'est pas nécessaire d'équiper l'individu d'un filtre. Si ce temps de récupération est compris entre 30 secondes et une minute il peut être utile de proposer un filtre à l'individu et si le temps est supérieur à une minute, il semble nécessaire de proposer un filtre.

D'autres examens plus précis peuvent être envisagés pour confirmer la nécessité d'un filtre.

Le résultat du test d'éblouissement permet de déterminer l'utilité de la présence d'un filtre lumineux intégré à l'équipement d'aide à la vision pour limiter l'éblouissement et le cas échéant de déterminer les caractéristiques de ce filtre.

## Test d'étendue du champ de vision

Il s'agit de déterminer si le champ de vision est limité par la présence d'une zone « aveugle » appelée scotome, par un test de vision périmétrique avec une cible statique ou dynamique, de forme et de niveaux de luminance variables.

Ce test est réalisé par l'affichage d'une série de cibles dont la taille et le contraste sont ajustés en fonction du résultat des tests précédents pour que l'individu puisse les observer sans difficulté. Le champ visuel décrit une cartographie des zones de perception du patient selon sa sensibilité lumineuse.

On détermine par exemple le champ de vision de l'individu en testant chaque oeil de cet individu avec une tablette dont les dimensions sont de l'ordre de 20 centimètres de large et de 30 centimètres de long, disposée à une distance de lecture de 40 centimètres de l'individu, et un angle au sommet de l'œil de +/-20 degrés. On peut également mesurer, pour une tablette de dimensions données l'angle maximal de vision de l'individu, comme cela est décrit par exemple dans le document US 7,549,743.

L'analyse de la cartographie et des caractéristiques du champ visuel de l'individu peut être exploitée pour optimiser les caractéristiques de l'équipement proposé à cet individu, tel le champ de vision de l'équipement, le besoin de rehaussement de contrastes, l'ajout de filtre et d'éclairage additionnel.

### Test d'oculomotricité

Ce test permet de déterminer si les yeux du porteur se dirigent avec la même agilité dans toutes les directions.

Pour cela, on peut utiliser un test de lecture d'un texte ou de suivi d'une cible.

Le test d'oculomotricité permet aussi de déterminer la stabilité de la fixation oculaire. Une cible présentant par exemple la forme d'une croix et de dimensions et contrastes adaptés à l'acuité visuelle du porteur est affichée au centre de l'écran. Une caméra enregistre et suit les mouvements du centre de l'œil, et plus particulièrement le reflet cornéen lumineux provoqué par la présence d'une lumière ponctuelle, par exemple, une diode électroluminescente, dirigée sur l'oeil selon une technique de suivi du regard connue en soi. La qualité de la fixation oculaire est évaluée en demandant à la personne de fixer le centre de croix pendant 30 secondes. Une caméra enregistre les variations de position du reflet cornéen durant le test. On détermine ainsi des mouvements continus ou discontinus de l'oeil.

La mesure peut être effectuée en vision monoculaire ou binoculaire.

L'évaluation de la qualité oculomotrice a un impact dans le choix de l'aide, et plus spécifiquement concernant le champ visuel associé à l'équipement. Un porteur avec une grande instabilité de fixation oculaire aura un meilleur confort et une meilleure performance avec un équipement présentant un grand champ de vision pour l'individu. Un équipement du type télé-agrandisseur serait par exemple dans un tel cas préférable à un équipement du type système de Galilée car présentant un champ de vision bien plus étendu.

### Test de vision des couleurs

Le test de la vision des couleurs a pour but de déterminer si la vision de l'individu comporte des défauts de perception des couleurs.

Le test consiste à classer plusieurs pastilles de différentes couleurs selon trois axes colorimétriques. Ce test de classement est réalisé par l'affichage de pastilles de couleurs sur l'écran que l'individu peut déplacer en touchant l'écran pour les classer dans l'ordre de leur tonalité à partir d'une couleur donnée. Seule la tonalité de pastilles affichées varie, la saturation et la luminosité des pastilles sont les mêmes. Les dyschromatopsies Rouge/Vert et Jaune/Bleu peuvent ainsi être détectées.

Les tests décrits précédemment sont réalisés les uns après les autres. Comme expliqué précédemment, le premier test réalisé est le test d'acuité visuelle. La taille des signes affichés ultérieurement est ainsi déterminée en fonction du résultat de ce test pour que l'individu puisse voir les signes facilement. On réalise ensuite de préférence le test d'acuité de lecture, puis le test d'oculomotricité, le test de pointage, le test de sensibilité au contraste, le test d'étendue du champ de vision et le test d'éblouissement, dans cet ordre.

### Etape b)

A l'étape b), les moyens informatiques déterminent au moins une utilisation du groupe d'au moins un équipement d'aide à la vision souhaitée par l'individu.

On entend par utilisation souhaitée à la fois les activités que l'individu souhaite réaliser avec l'équipement et les conditions ergonomiques d'utilisation souhaitées de l'équipement.

En pratique on détermine à l'étape b) si l'une au moins des utilisations souhaitées par l'individu est souhaitée :

- utilisation en lecture,
- utilisation en écriture,
- utilisation pour voir de loin,
- utilisation pour voir de près,
- utilisation pour regarder la télévision,
- utilisation pour travailler sur un écran situé à une distance intermédiaire.

On entend par vision de loin pour une personne malvoyante la visualisation d'un objet situé à une distance de l'individu supérieure ou égale à 2 mètres.

On entend par vision de près la visualisation d'un objet situé à une distance de l'individu comprise entre 30 et 50 centimètre, par exemple égale à 40 centimètres.

On entend par distance intermédiaire une distance de travail sur écran ergonomique pour l'individu. Cette distance est généralement comprise entre 50 centimètres et un mètre.

Pour cela, le dispositif enregistre, pour chaque utilisation possible prise en compte par les moyens informatiques, un indice d'importance de cette utilisation pour l'individu. Par exemple, l'individu donne un indice d'importance compris entre 1 et 5 à chacune des utilisations proposées ci-dessus.

De préférence, à l'étape b), l'une au moins des conditions d'ergonomie d'utilisation suivantes souhaitées par l'individu pour les utilisations envisagées est déterminée :

- utilisation en extérieur,
- utilisation en intérieur,
- utilisation avec les mains libres,
- utilisation pendant une longue durée.

On entend par utilisation pendant une longue durée une utilisation supérieure à quelques minutes, par exemple une utilisation supérieure à 30 minutes.

Le dispositif enregistre, de manière globale pour toutes les utilisations souhaitées, les caractéristiques ergonomiques souhaitées pour l'équipement d'aide à la vision. Pour cela le dispositif enregistre un paramètre ergonomique binaire, par exemple 1 ou 0, + ou -, indiquant si la condition d'ergonomie considérée doit être ou non remplie par l'équipement d'aide à la vision pour satisfaire l'individu.

En variante, on peut envisager que le dispositif enregistre des paramètres ergonomiques différents pour chaque utilisation de l'équipement.

Le dispositif enregistre par exemple si l'individu souhaite garder les mains libres ou non. Il enregistre également si la durée d'utilisation envisagée est longue, par exemple pour lire un livre pendant plusieurs heures, ou si elle est courte, par exemple pour lire les emballages des produits au supermarché.

Etape c)

Les moyens informatiques de la tablette comportent en mémoire une liste préétablie d'équipements d'aide à la vision.

Ces équipements d'aide à la vision sont répartis en sept catégories d'équipements d'aide à la vision.

Une première catégorie comprend les loupes.

La loupe est un produit très simple permettant d'agrandir un texte ou un objet. Le choix d'une loupe est un compromis entre grossissement et champ de vision afin d'offrir une bonne vitesse de lecture.

Il existe différents types de loupes, parmi lesquels par exemple:

- les loupes de poche pour une durée d'utilisation courte, en intérieur comme en extérieur,
- les loupes à main pour un usage quotidien, une durée d'utilisation courte ou longue, en intérieur comme en extérieur,
- les loupes à poser qui occasionnent moins de fatigue, pour une durée d'utilisation plus longue et pour garder les mains libres,
- les loupes éclairantes qui associent grossissement et fort contraste.

Une deuxième catégorie contient les lunettes microscopiques. Ces lunette sont composées d'une monture et de lentilles ophtalmiques de fortes puissances, par exemple de puissance comprise entre +6 et +36 dioptries.

Une troisième catégorie contient les systèmes télescopiques de Galilée et Kepler. Ce sont des dispositifs destinés à être montés sur une paire de lunettes. Ils sont destinés à la vision de près ou de loin. Ils permettent à la personne malvoyante d'effectuer tous types de travaux en gardant les mains libres dans la plupart des cas.

Ces dispositifs peuvent présenter une large gamme de grossissement et apportent un large champ de vision.

Enfin, pour une utilisation ponctuelle, par exemple lire un nom de rue, les dispositifs monoculaires à main sauront apporter l'aide nécessaire tout en restant discrets.

Une quatrième catégorie comprend les aides électroniques portables. Les aides électroniques présentent de très forts grossissements et permettent d'améliorer la perception des contrastes. Elles sont simples d'utilisation.

Les aides électroniques portables sont légères et compactes. Elles permettent d'apporter une grande autonomie dans la vie quotidienne.

Les aides électroniques transportables se connectent sur tous types d'écrans et peuvent être ainsi déplacées facilement.

Une cinquième catégorie comprend les aides électroniques du type télé-agrandisseurs.

Une sixième catégorie comprend les lampes. Un éclairage approprié et de bonne qualité augmente significativement l'acuité visuelle.

Les différentes températures de couleurs disponibles permettent ainsi de définir l'éclairage le plus approprié et le plus confortable. Cette optimisation de l'éclairage permet de réduire le grossissement des autres équipements d'aide nécessaires.

Enfin, la septième catégorie contient les filtres.

Ils permettent une amélioration de la perception des contrastes et une diminution de l'éblouissement.

Comme les lampes, les filtres ne présentent pas de grossissement et peuvent avantageusement être associés à un autre équipement d'aide à la vision.

Chaque équipement d'aide à la vision est répertorié dans une liste sous la forme d'un registre électronique mis en mémoire dans les moyens informatiques de la tablette.

Chaque enregistrement de ce registre comprend un identifiant de l'équipement, des premiers indices de performance fonction de l'aide apportée par cet équipement pour toutes les caractéristiques de vision possibles de l'individu et des seconds indices de performance fonction de l'adaptation de cet équipement à toutes les utilisations possibles.

L'identifiant de l'équipement d'aide à la vision correspond par exemple à son nom, à une référence du fabricant ou à un code regroupant plusieurs informations, par exemple référence du fabricant et catégorie d'aide à la vision à laquelle il appartient.

De préférence, chaque enregistrement du registre électronique comporte en outre un indicateur des conditions ergonomiques d'utilisation de l'équipement correspondant.

Les premier et deuxième indices de performances et l'indicateur des conditions ergonomiques d'utilisation sont déterminés empiriquement lors d'une phase d'initialisation du registre. Ils peuvent être modifiés sur la base de l'expérience de l'utilisateur du dispositif, en fonction notamment des retours donnés par les individus utilisant les équipements d'aide à la vision.

Les premiers indices de performances de l'équipement dépendent essentiellement des caractéristiques optiques de l'équipement, par exemple :

- grossissement de l'équipement,
- capacités d'amélioration du contraste,
- étendue du champ de vision avec l'équipement,
- possibilité d'utilisation avec une lampe ou un filtre,
- distance de focalisation courte ou intermédiaire, fixe ou variable,
- profondeur de champ de l'équipement.

En pratique les premiers indices de performance de chaque équipement peuvent par exemple comprendre un premier ensemble d'indices comprenant tous les grossissements possibles de l'équipement. L'enregistrement du registre correspondant à un équipement d'aide à la vision pouvant avoir un grossissement compris entre 5 et 10 comprendra ainsi les premiers indices 5, 6, 7, 8, 9 et 10 pour le grossissement.

Ces premiers indices comprennent également un indice binaire indiquant si l'équipement est adapté à améliorer la perception des contrastes : les équipements d'aide à la vision électroniques sont par exemple adaptés à réaliser cette amélioration, et auront donc l'indice 1 pour l'amélioration du contraste. Ces équipements d'aide à la vision électroniques auront également un indice 1 pour le grossissement.

Les équipements optiques du type loupe n'étant pas adaptés à améliorer le contraste, ils auront l'indice 0 pour l'amélioration du contraste.

Les deuxièmes indices de performances de l'équipement dépendent des caractéristiques techniques décrites précédemment et d'autres caractéristiques pratiques de l'équipement, qui le rendent plus ou moins apte à être utilisé pour une utilisation donnée par exemple :

- possibilité de transporter l'équipement sur soi, ou au contraire
- possibilité de fixer l'équipement sur un support fixe,
- possibilité de fixer l'équipement sur une paire de lunettes,
- distance d'utilisation de l'équipement.

Les deuxièmes indices de performance correspondent par exemple à une note sur cinq valeurs, par exemple variant de 0 à 4, des performances de l'équipement pour chaque utilisation.

Une loupe à poser éclairante sera particulièrement bien adaptée à être utilisée pour lire. Son indice de performance pour la lecture sera donc égal à quatre.

En revanche, cette loupe sera peu adaptée à une utilisation pour écrire. Son indice de performance pour l'écriture sera donc égal à 0.

Les loupes n'étant pas adaptées à la vision de loin et intermédiaire, les seconds indices de performance de cette loupe pour les utilisations en vision de loin, pour regarder la télévision et pour travailler à une distance intermédiaire seront égaux à 0.

L'indicateur d'ergonomie d'utilisation est ici un indicateur binaire donnant l'adaptation de l'équipement à certaines conditions d'utilisation.

Par exemple, un équipement lourd et tenu à la main ne sera pas par exemple adapté à une utilisation longue. Un équipement laissant accès à un champ de vision très limité, ou présentant une profondeur de champ très faible, donc difficile à maintenir à sa distance d'utilisation ne seront pas adaptés à une utilisation longue. Ces équipements auront par exemple un indicateur de valeur 0 pour l'adaptation à une utilisation de longue durée.

Au contraire, un équipement d'aide à la vision pouvant être utilisé à une distance ergonomique et posé sur un support autorise une utilisation de plus longue durée et aura un indicateur égal à 1 pour cette condition.

Un équipement devant être connecté au secteur ne sera pas adapté à une utilisation à l'extérieur. Les équipements de petites taille, léger et peu encombrant, comme certaines loupes par exemple, qui ne nécessitent pas d'alimentation électrique seront parfaitement adaptés à être utilisés à l'extérieur.

Par exemple, dans le cas de la loupe décrite précédemment, en supposant que la loupe à poser éclairante soit de petite taille et fonctionne sur batterie, celle-ci sera adaptée à une durée d'utilisation courte ou longue, puisqu'elle peut être posée et à une utilisation à l'extérieur.

Selon l'exemple de réalisation décrit ici, les moyens informatiques sont programmés pour présélectionner, au cours d'un premier tri, parmi la liste préétablie d'équipements d'aide à la vision, c'est-à-dire parmi les différents enregistrements du registre, un ensemble d'équipements d'aide à la vision adaptés à aider l'individu en fonction d'au moins une caractéristique de sa vision déterminée à l'étape a).

Par exemple, les moyens informatiques sélectionnent dans le registre uniquement les équipements adaptés à fournir le grossissement nécessaire correspondant à l'acuité fonctionnelle déterminée précédemment par le test d'acuité en lecture.

Si le test d'acuité fonctionnel a montré que l'individu a une acuité fonctionnelle de 1/10, le grossissement de l'équipement adapté est égal à 6.

Le premier tri sélectionne alors tous les équipements dont les premiers indices correspondant aux grossissements possibles contiennent la valeur 6. Ces équipements forment l'ensemble d'équipements déterminé à l'étape c1).

Lors d'une deuxième étape de tri, les moyens informatiques sont ici programmés pour sélectionner, parmi cet ensemble d'équipements d'aide à la vision, un sous-ensemble d'équipements d'aide à la vision présentant les conditions d'ergonomie d'utilisation souhaitées par l'individu.

Lors de ce deuxième tri, les moyens informatiques sélectionnent ici les équipements dont les indicateurs concernant les conditions d'utilisations correspondent aux paramètres ergonomiques de l'utilisation souhaitée par l'individu.

Par exemple, si l'individu a indiqué qu'il souhaitait utiliser l'équipement sur une longue durée, en intérieur et avec les mains libres, le sous-ensemble d'équipement déterminé lors du deuxième tri comporte tous les équipements de l'ensemble déterminé au premier tri pouvant être utilisés dans ces conditions.

Lors d'un troisième tri, les moyens informatiques du dispositif sélectionnent parmi le sous-ensemble déterminé au deuxième tri, les équipements d'aide à la vision dont l'un au moins des seconds indices de performance est supérieur ou égal à l'indice d'importance de l'utilisation correspondante attribué par l'individu.

Par exemple, si l'individu a attribué l'indice 4 à l'utilisation en lecture, 2 à l'utilisation en écriture et 0 à toutes les autres utilisations, le troisième tri sélectionne les équipements dont le deuxième indice de performance pour la lecture est égal à 4 et tous ceux dont le deuxième indice de performance pour l'écriture est égal à 2, 3, ou 4.

Enfin, pour chaque équipement sélectionné lors du troisième tri, les moyens informatiques déterminent un score de performance en fonction des deuxièmes indices de performance de chaque équipement pour chaque utilisation et en fonction de l'indice d'importance attribuée à chaque utilisation par l'individu.

Selon un premier mode de réalisation, le score de performance est un score global obtenu en sommant les deuxièmes indices de performances de chaque équipement pour toutes les utilisations dont l'indice d'importance pour l'individu est supérieur à une valeur seuil, par exemple supérieur à 2.

Le dispositif selon l'invention affiche alors le groupe d'équipement ainsi déterminé sous forme d'une liste classée en fonction de ce score global, par exemple selon les scores globaux décroissants.

Selon un deuxième mode de réalisation, le score de performance déterminé est un score pondéré reflétant de manière plus précise l'adéquation entre les performances de l'équipement pour les utilisations possibles de l'équipement et l'importance de ces utilisations pour l'individu.

Pour chacune des utilisations envisagées, les moyens informatiques calculent alors un ratio entre le deuxième indice de performance de l'équipement correspondant à cette utilisation et l'indice d'importance de cette utilisation pour l'individu. Les ratios de toutes les utilisations sont ensuite sommés pour obtenir le score pondéré de l'équipement.

En pratique, le dispositif affiche la liste des équipements ainsi déterminés classés selon des scores pondérés décroissants. Le dispositif peut également transmettre la liste déterminée à un ordinateur.

En variante, le dispositif détermine un unique équipement correspondant à l'équipement ayant le plus grand score global ou pondéré.

De nombreuses variantes peuvent être envisagées. L'enchaînement des étapes de tri peut notamment être modifié. On peut par exemple pendre d'abord en compte les utilisations envisagées par l'individu avant de sélectionner les équipements présentant les conditions ergonomiques demandées par l'individu. A chaque étape de tri, la sélection est effectuée parmi les équipements sélectionnés à l'étape précédente.

On peut également envisager toute manière connue de l'homme du métier pour déterminer un score de performance pour chaque équipement : tout type de moyenne, moyenne pondérée etc...

On entend ici par groupe d'au moins un équipement un groupe comportant un ou plusieurs équipements appartenant à une ou plusieurs catégories d'équipement.

Selon une variante simplifiée de l'invention, on peut envisager que le groupe d'équipements déterminé par le dispositif corresponde à une des sept catégories d'équipements décrites précédemment. Pour cela, à l'issue de l'un des tris décrits précédemment, le dispositif calcule un score de performance associé à chaque catégorie d'équipements présentant des équipements sélectionnés par le tri précédent. Ce score de catégorie est par exemple égal à la moyenne sur tous les équipements de cette catégorie des scores globaux ou des scores pondérés décrits précédemment. Le dispositif affiche alors la catégorie présentant le meilleur score, ou affiche un classement des catégories par scores décroissants.

En variante, on peut évidemment envisager que le dispositif selon l'invention soit constitué par une tablette non tactile, un écran d'ordinateur ou de téléphone ou encore qu'il comporte des moyens de projection sur un écran. On peut en outre prévoir que le dispositif comporte des moyens d'ajustement de la taille des signes affichés en fonction de la distance de l'individu par rapport à l'écran d'affichage.

D'autres types de test de la vision peuvent être envisagés, ainsi que des tests psychosensoriels, par exemple pour évaluer la perception des reliefs avec des tests stéréoscopiques, ce qui peut avoir une influence sur le choix du filtre.

Des tests pour déterminer l'œil dominant de l'individu, la coordination œil-tête ou des tests d'ergonomie des équipements peuvent également être réalisés.

Les résultats de ces tests ainsi que les résultats des tests de pointage, d'éblouissement, de champ de vision, de perception des couleurs et d'oculomotricité sont pris en compte par les moyens informatiques soit au cours des étapes décrites précédemment, afin d'affiner la sélection des équipements par la prise en compte de ces informations, soit à la fin du processus de sélection, afin d'ajuster le score de performance des équipements et permettre d'optimiser leur classement.

Ces informations peuvent également être prises en compte afin de déterminer s'il est opportun d'ajouter à l'équipement sélectionné par les étapes décrites précédemment un deuxième équipement du type filtre ou éclairage additionnel.

## Revendications

1. Dispositif (10) pour déterminer un groupe d'au moins un équipement d'aide à la vision adapté à la vision d'un individu, comportant des moyens informatiques comportant en mémoire une liste préétablie d'équipements d'aide à la vision se présentant sous la forme d'un registre électronique dont chaque enregistrement comporte :

   - un identifiant de chaque équipement d'aide à la vision,
   - des premiers indices de performance fonction de l'aide apportée par cet équipement pour toutes les caractéristiques de vision possibles de l'individu et
   - des seconds indices de performance fonction de l'adaptation de cet équipement à toutes les utilisations possibles,
   - un indicateur d'au moins une condition ergonomique d'utilisation de l'équipement correspondant, et étant programmés pour :

      a) déterminer au moins une caractéristique de la vision dudit individu,
      b) déterminer au moins une utilisation du groupe d'au moins un équipement d'aide à la vision souhaitée par l'individu et au moins une condition d'ergonomie souhaitée par l'individu pour l'utilisation souhaitée, parmi les conditions d'ergonomie suivantes : utilisation en intérieur, utilisation en extérieur, distance d'utilisation ergonomique standard, durée d'utilisation longue ou courte,
      c) déterminer ledit groupe d'au moins un équipement d'aide à la vision en fonction de la caractéristique de la vision, de la condition d'ergonomie et de l'utilisation souhaitée par l'individu en effectuant les étapes suivantes :

         - dans une étape c1), présélectionner parmi cette liste préétablie, un ensemble d'équipements d'aide à la vision adaptés à aider l'individu en fonction d'au moins la caractéristique de sa vision déterminée à l'étape a), les moyens informatiques étant programmés pour présélectionner dans ledit registre l'ensemble des équipements d'aide à la vision pour lesquels au moins un desdits premiers indices correspond à la caractéristique de vision de l'individu associée,
         - dans une étape c2), sélectionner parmi ledit ensemble d'équipements d'aide à la vision déterminé à l'étape c1), un sous-ensemble d'équipements d'aide à la vision présentant les conditions d'ergonomie d'utilisation souhaitées par l'individu, les moyens informatiques étant programmés pour sélectionner dans ledit ensemble déterminé à l'étape c1), le sous-ensemble d'équipements d'aide à la vision pour lesquels au moins un indicateur des conditions ergonomiques d'utilisation de l'équipement correspond à la condition d'utilisation souhaitée par l'individu,
         - dans une étape c3), sélectionner parmi ledit sous-ensemble d'équipements d'aide à la vision déterminé à l'étape c2), un sous-ensemble d'équipements d'aide à la vision le mieux adapté aux utilisations souhaitées par l'individu, les moyens informatiques étant programmés pour sélectionner, dans ledit ensemble déterminé à l'étape c2), le sous-ensemble d'équipements d'aide à la vision pour lesquels le second indice est supérieur à une valeur seuil pour au moins une utilisation souhaitée par l'individu.

2. Dispositif (10) selon la revendication 1, dans lequel, à l'étape a), l'une au moins des caractéristiques suivantes de la vision de l'individu est déterminée : acuité visuelle, acuité en lecture, sensibilité au contraste, étendue du champ de vision, éblouissement, oculomotricité, coordination œil-main, vision des couleurs, stéréoscopie.

3. Dispositif (10) selon l'une des revendications 1 et 2, comportant une partie d'affichage (11) et dans lequel, à l'étape a), la caractéristique de la vision de l'individu est déterminée grâce à un test de reconnaissance d'une image (20) affichée sur ladite partie d'affichage (11).

4. Dispositif (10) selon la revendication 3, dans lequel, à l'étape a), plusieurs caractéristiques de la vision de l'individu sont déterminées successivement et dans lequel le contenu et/ou l'enchaînement des tests de la vision ultérieurs est adapté en fonction des résultats des tests précédents.

5. Dispositif (10) selon l'une des revendications 1 à 4, dans lequel, à l'étape b), l'une au moins des utilisations suivantes souhaitées par l'individu est déterminée : lire, écrire, voir de loin, voir de près, regarder la télévision, travailler sur un objet situé à une distance intermédiaire.

**6.** Dispositif (10) selon l'une des revendications 1 à 5, dans lequel, à l'étape b), le dispositif enregistre, pour chaque utilisation souhaitée, un indice d'importance de cette utilisation pour l'individu.

**7.** Dispositif selon l'une des revendications précédentes, adapté à la détermination d'un groupe d'au moins un équipement d'aide à la vision pour un individu malvoyant.

**8.** Procédé pour déterminer un groupe d'au moins un équipement d'aide à la vision adapté à la vision d'un individu, mettant en oeuvre des moyens informatiques comportant en mémoire une liste préétablie d'équipements d'aide à la vision se présentant sous la forme d'un registre électronique dont chaque enregistrement comporte :

    - un identifiant de chaque équipement d'aide à la vision,
    - des premiers indices de performance fonction de l'aide apportée par cet équipement pour toutes les caractéristiques de vision possibles de l'individu et
    - des seconds indices de performance fonction de l'adaptation de cet équipement à toutes les utilisations possibles,
    - un indicateur d'au moins une condition ergonomique d'utilisation de l'équipement correspondant,

ledit procédé comportant les étapes suivantes réalisées par lesdits moyens informatiques

    a) déterminer au moins une caractéristique de la vision dudit individu,
    b) déterminer au moins une utilisation du groupe d'au moins un équipement d'aide à la vision souhaitée par l'individu et au moins une condition d'ergonomie souhaitée par l'individu pour l'utilisation souhaitée, parmi les conditions d'ergonomie suivantes : utilisation en intérieur, utilisation en extérieur, distance d'utilisation ergonomique standard, durée d'utilisation longue ou courte,
    c) déterminer ledit groupe d'au moins un équipement d'aide à la vision en fonction de la caractéristique de la vision, de la condition d'ergonomie et de l'utilisation souhaitée par l'individu en effectuant les étapes suivantes :

        - dans une étape c1), présélectionner parmi cette liste préétablie, un ensemble d'équipements d'aide à la vision adaptés à aider l'individu en fonction d'au moins la caractéristique de sa vision déterminée à l'étape a), les moyens informatiques étant programmés pour présélectionner dans ledit registre l'ensemble des équipements d'aide à la vision pour lesquels au moins un desdits premiers indices correspond à la caractéristique de vision de l'individu associée,
        - dans une étape c2), sélectionner parmi ledit ensemble d'équipements d'aide à la vision déterminé à l'étape c1), un sous-ensemble d'équipements d'aide à la vision présentant les conditions d'ergonomie d'utilisation souhaitées par l'individu, les moyens informatiques étant programmés pour sélectionner dans ledit ensemble déterminé à l'étape c1), le sous-ensemble d'équipements d'aide à la vision pour lesquels au moins un indicateur des conditions ergonomiques d'utilisation de l'équipement correspond à la condition d'utilisation souhaitée par l'individu,
        - dans une étape c3), sélectionner parmi ledit sous-ensemble d'équipements d'aide à la vision déterminé à l'étape c2), un sous-ensemble d'équipements d'aide à la vision le mieux adapté aux utilisations souhaitées par l'individu, les moyens informatiques étant programmés pour sélectionner, dans ledit ensemble déterminé à l'étape c2), le sous-ensemble d'équipements d'aide à la vision pour lesquels le second indice est supérieur à une valeur seuil pour au moins une utilisation souhaitée par l'individu.

**Patentansprüche**

**1.** Vorrichtung (10) zur Bestimmung einer Gruppe von mindestens einem für das Sehvermögen einer Person geeigneten Sehhilfegerät, die Informatikmittel aufweist, die in einem Speicher eine vorab erstellte Liste von Sehhilfegeräten in Form eines elektronischen Registers enthalten, wobei jeder Datensatz Folgendes aufweist:

    - eine Kennung für jedes Sehhilfegerät,
    - erste Leistungsindizes, die von der durch dieses Gerät geleisteten Unterstützung für alle möglichen Merkmale des Sehvermögens der Person abhängen, und
    - zweite Leistungsindizes, die von der Eignung dieses Geräts für alle möglichen Verwendungen abhängen,
    - einen Indikator für mindestens eine ergonomische Bedingung für die Verwendung des entsprechenden Geräts, und die programmiert sind, um:

        a) mindestens ein Merkmal des Sehvermögens der Person zu bestimmen,
        b) mindestens eine von der Person gewünschte Verwendung der Gruppe von mindestens einem Sehhilfegerät und mindestens eine von der Person für die gewünschte Verwendung gewünschte ergonomischen Bedingung aus den folgenden ergonomischen Bedingungen zu bestimmen: Verwendung in Innenräumen, Verwendung im Freien, ergonomische Standard-Verwendungsentfernung, lange oder kurze Verwendungsdauer,
c) die Gruppe von mindestens einem Sehhilfegerät entsprechend dem Merkmal des Sehvermögens, der ergonomischen Bedingung und der von der Person gewünschten Verwendung durch Ausführen der folgenden Schritte zu bestimmen:

- in einem Schritt c1) aus dieser vorab erstellten Liste eine Menge von Sehhilfegeräten vorab auswählen, die geeignet sind, die Person entsprechend mindestens dem in Schritt a) ermittelten Merkmal des Sehvermögens zu unterstützen, wobei die Informatikmittel so programmiert sind, dass sie in dem Register die Menge von Sehhilfegeräten vorab auswählen, bei denen mindestens einer der genannten ersten Indizes mit dem Merkmal des Sehvermögens der betreffenden Person übereinstimmt,
- in einem Schritt c2) aus der in Schritt c1) bestimmten Menge von Sehhilfegeräten eine Untermenge von Sehhilfegeräten auswählen, die die von der Person gewünschten ergonomischen Bedingungen für die Verwendung erfüllen, wobei die Informatikmittel so programmiert sind, dass sie aus der in Schritt c1) bestimmten Menge die Untermenge von Sehhilfegeräten auswählen, bei denen mindestens ein Indikator der ergonomischen Bedingungen für die Verwendung des Geräts der von der Person gewünschten Bedingung für die Verwendung entspricht,
- in einem Schritt c3) aus der in Schritt c2) bestimmten Untermenge von Sehhilfegeräten eine Untermenge von Sehhilfegeräten auswählen, die den von der Person gewünschten Verwendungen am besten entspricht, wobei die Informatikmittel so programmiert sind, dass sie aus der in Schritt c2) bestimmten Menge die Untermenge von Sehhilfegeräten auswählen, bei denen der zweite Index für mindestens eine von der Person gewünschte Verwendung über einem Schwellenwert liegt.

2. Vorrichtung (10) nach Anspruch 1, wobei in Schritt a) mindestens eines der folgenden Merkmale des Sehvermögens der Person bestimmt wird: Sehschärfe, Leseschärfe, Kontrastempfindlichkeit, Umfang des Gesichtsfeldes, Blendung, Augenmotorik, Hand-Augen-Koordination, Farbwahrnehmung, Stereoskopie.

3. Vorrichtung (10) nach einem der Ansprüche 1 und 2, die einen Anzeigebereich (11) aufweist, über den in Schritt a) das Merkmal des Sehvermögens der Person anhand eines Tests zur Erkennung eines auf dem Anzeigebereich (11) angezeigten Bildes (20) bestimmt wird.

4. Vorrichtung (10) nach Anspruch 3, wobei in Schritt a) mehrere Merkmale des Sehvermögens der Person nacheinander bestimmt werden und wobei der Inhalt und/oder die Abfolge der nachfolgenden Sehtests entsprechend den Ergebnissen der vorherigen Tests angepasst wird.

5. Vorrichtung (10) nach einem der Ansprüche 1 bis 4, wobei in Schritt b) mindestens eine der folgenden von der Person gewünschten Verwendungen bestimmt wird: Lesen, Schreiben, Sehen in der Ferne, Sehen in der Nähe, Fernsehen, Arbeiten an einem Objekt in mittlerer Entfernung.

6. Vorrichtung (10) nach einem der Ansprüche 1 bis 5, wobei die Vorrichtung in Schritt b) für jede gewünschte Verwendung einen Index der Wichtigkeit dieser Verwendung für die Person speichert.

7. Vorrichtung nach einem der vorhergehenden Ansprüche, die zur Bestimmung einer Gruppe von mindestens einem Sehhilfegerät für eine sehbehinderte Person geeignet ist.

8. Verfahren zur Bestimmung einer Gruppe von mindestens einem für das Sehvermögen einer Person geeigneten Sehhilfegerät, das Informatikmittel verwendet, die in einem Speicher eine vorab erstellte Liste von Sehhilfegeräten in Form eines elektronischen Registers enthalten, wobei jeder Datensatz Folgendes aufweist:

- eine Kennung für jedes Sehhilfegerät,
- erste Leistungsindizes, die von der durch dieses Gerät geleisteten Unterstützung für alle möglichen Merkmale des Sehvermögens der Person abhängen, und
- zweite Leistungsindizes, die von der Eignung dieses Geräts für alle möglichen Verwendungen abhängen,

- einen Indikator für mindestens eine ergonomische Bedingung für die Verwendung des entsprechenden Geräts, wobei das Verfahren die folgenden Schritte aufweist, die von den Informatikmitteln ausgeführt werden, um:

a) mindestens ein Merkmal des Sehvermögens der Person zu bestimmen,
b) mindestens eine von der Person gewünschte Verwendung der Gruppe von mindestens einem Sehhilfegerät und mindestens eine von der Person für die gewünschte Verwendung gewünschte ergonomischen Bedingung aus den folgenden ergonomischen Bedingungen zu bestimmen: Verwendung in Innenräumen, Verwendung im Freien, ergonomische Standard-Verwendungsentfernung, lange oder kurze Verwendungsdauer,
c) die Gruppe von mindestens einem Sehhilfegerät entsprechend dem Merkmal des Sehvermögens, der ergonomischen Bedingung und der von der Person gewünschten Verwendung durch Ausführen der folgenden Schritte zu bestimmen:

- in einem Schritt c1) aus dieser vorab erstellten Liste eine Menge von Sehhilfegeräten vorab auswählen, die geeignet sind, die Person entsprechend mindestens dem in Schritt a) ermittelten Merkmal des Sehvermögens zu unterstützen, wobei die Informatikmittel so programmiert sind, dass sie in dem Register die Menge von Sehhilfegeräten vorab auswählen, bei denen mindestens einer der genannten ersten Indizes mit dem Merkmal des Sehvermögens der betreffenden Person übereinstimmt,
- in einem Schritt c2) aus der in Schritt c1) bestimmten Menge von Sehhilfegeräten eine Untermenge von Sehhilfegeräten auswählen, die die von der Person gewünschten ergonomischen Bedingungen für die Verwendung erfüllen, wobei die Informatikmittel so programmiert sind, dass sie aus der in Schritt c1) bestimmten Menge die Untermenge von Sehhilfegeräten auswählen, bei denen mindestens ein Indikator der ergonomischen Bedingungen für die Verwendung des Geräts der von der Person gewünschten Bedingung für die Verwendung entspricht,
- in einem Schritt c3) aus der in Schritt c2) bestimmten Untermenge von Sehhilfegeräten eine Untermenge von Sehhilfegeräten auswählen, die den von der Person gewünschten Verwendungen am besten entspricht, wobei die Informatikmittel so programmiert sind, dass sie aus der in Schritt c2) bestimmten Menge die Untermenge von Sehhilfegeräten auswählen, bei denen der zweite Index für mindestens eine von der Person gewünschte Verwendung über einem Schwellenwert liegt.

**Claims**

1. Device (10) for determining a group of at least one vision aid apparatus suitable for the vision of an individual, comprising computing means comprising in memory a pre-established list of vision aid apparatuses in the form of an electronic register, each record of which comprises:

- an identifier of each vision aid apparatus,
- first performance indices as a function of the aid provided by this apparatus for all possible vision characteristics of the individual and
- second performance indices as a function of the suitability of this apparatus for all possible uses,
- an indicator of at least one ergonomic condition of use of the corresponding apparatus, and programmed to:

a) determine at least one characteristic of the vision of said individual,
b) determine at least one use of the group of at least one vision aid apparatus desired by the individual and at least one ergonomic condition desired by the individual for the desired use, from among the following ergonomic conditions: use indoors, use outdoors, standard ergonomic use distance, long or short duration of use,
c) determine said group of at least one vision aid apparatus as a function of the characteristic of the vision, of the ergonomic condition and of the use desired by the individual by carrying out the following steps:

- in a step c1), preselecting, from among this pre-established list, a set of vision aid apparatuses suitable for aiding the individual as a function of at least the characteristic of their vision determined in step a), the computing means being programmed to preselect, from said register, all of the vision aid apparatuses for which at least one of said first indices corresponds to the associated vision characteristic of the individual,

- in a step c2), selecting, from among said set of vision aid apparatuses that is determined in step c1), a subset of vision aid apparatuses exhibiting the ergonomic conditions of use desired by the individual, the computing means being programmed to select, from said set determined in step c1), the subset of vision aid apparatuses for which at least one indicator of the ergonomic conditions of use of the apparatus corresponds to the condition of use desired by the individual,

- in a step c3), selecting, from among said subset of vision aid apparatuses that is determined in step c2), a subset of vision aid apparatuses that is most suitable for the uses desired by the individual, the computing means being programmed to select, from said set determined in step c2), the subset of vision aid apparatuses for which the second index is greater than a threshold value for at least one use desired by the individual.

2. Device (10) according to Claim 1, wherein, in step a), at least one of the following characteristics of the vision of the individual is determined: visual acuity, reading acuity, sensitivity to contrast, extent of the field of vision, glare, oculomotricity, eye-hand coordination, colour vision, stereoscopy.

3. Device (10) according to one of Claims 1 and 2, comprising a display part (11) and wherein, in step a), the characteristic of the vision of the individual is determined by virtue of a test of recognition of an image (20) displayed on said display part (11).

4. Device (10) according to Claim 3, wherein, in step a), several characteristics of the vision of the individual are determined successively and wherein the content and/or the sequencing of the subsequent vision tests is adapted as a function of the results of the previous tests.

5. Device (10) according to one of Claims 1 to 4, wherein, in step b), at least one of the following uses desired by the individual is determined: reading, writing, far vision, near vision, watching television, working on an object situated at an intermediate distance.

6. Device (10) according to one of Claims 1 to 5, wherein, in step b), the device records, for each desired use, an index of importance of this use for the individual.

7. Device according to one of the preceding claims, suitable for the determination of a group of at least one vision aid apparatus for a partially sighted individual.

8. Method for determining a group of at least one vision aid apparatus suitable for the vision of an individual, implementing computing means comprising in memory a pre-established list of vision aid apparatuses in the form of an electronic register, each record of which comprises:

- an identifier of each vision aid apparatus,
- first performance indices as a function of the aid provided by this apparatus for all possible vision characteristics of the individual and
- second performance indices as a function of the suitability of this apparatus for all possible uses,
- an indicator of at least one ergonomic condition of use of the corresponding apparatus,

said method comprising the following steps carried out by said computing means:

a) determining at least one characteristic of the vision of said individual,
b) determining at least one use of the group of at least one vision aid apparatus desired by the individual and at least one ergonomic condition desired by the individual for the desired use, from among the following ergonomic conditions: use indoors, use outdoors, standard ergonomic use distance, long or short duration of use,
c) determining said group of at least one vision aid apparatus as a function of the characteristic of the vision, of the ergonomic condition and of the use desired by the individual by carrying out the following steps:

- in a step c1), preselecting, from among this pre-established list, a set of vision aid apparatuses suitable for aiding the individual as a function of at least the characteristic of their vision determined in step a), the computing means being programmed to preselect, from said register, all of the vision aid apparatuses for which at least one of said first indices corresponds to the associated vision characteristic of the individual,
- in a step c2), selecting, from among said set of vision aid apparatuses that is determined in step c1), a subset of vision aid apparatuses exhibiting the ergonomic conditions of use desired by the individual, the computing means being programmed to select, from said set determined in step c1), the subset of vision aid apparatuses for which at least one indicator of the ergonomic conditions of use of the apparatus corresponds to the condition of use desired by the individual,

- in a step c3), selecting, from among said subset of vision aid apparatuses that is determined in step c2), a subset of vision aid apparatuses that is most suitable for the uses desired by the individual, the computing means being programmed to select, from said set determined in step c2), the subset of vision aid apparatuses for which the second index is greater than a threshold value for at least one use desired by the individual.

Fig.1

11

10

12

Fig.3

10

11

O S Z D H
N D S Z V
S D N V Z
V Z N K H
S N H O S
H D O N R
K R H V C

20

12

Fig.4

10

11

15

H

16

18

19

17

Fig.2

100 — Caractérisation de la vision du porteur

110 — Acuité visuelle → Acuité fonctionnelle → Sensibilité contraste → ......

120 — Traitement des données

130 — Base de données calibrée des aides

150 — Conditions d'utilisations souhaitées

140 — 1ère sélection de l'équipement d'aide

160 — Sélection des paramètres des aides adaptées

250 — Activités souhaitées

170 — Analyse

180 — 2ème sélection de l'équipement d'aide

260 — Sélection des paramètres des aides adaptées

190 — Analyse

200 — Sélection finale de l'équipement d'aide

## RÉFÉRENCES CITÉES DANS LA DESCRIPTION

**Documents brevets cités dans la description**

- EP 1336924 A1
- EP 1333393 A1
- US 7549743 B

**Littérature non-brevet citée dans la description**

- **WILLIAM J. BENJAMIN**. Borish's Clinical Refraction. Butterworth-Heinemann/Elsevier, 2006